# EUROPÄISCHE PATENTANMELDUNG

(11) **EP 2 298 762 A2**
(43) Veröffentlichungstag der Anmeldung: **23.03.2011**
(21) Anmeldenummer: 10013111.9
(22) Anmeldetag: 29.03.2004
(51) Int. Cl.: C07D 405/06, C07D 405/12, C07D 405/14, C07D 413/06, C07D 413/12, C07D 413/14, A61K 31/538, A61K 31/453, A61K 31/4709, A61K 31/4725, A61P 31/04

(54) **Heterobicyclische Verbindungen mit antibakterieller Aktivität**

(30) Priorität: 08.04.2003 DE 10316081
(62) Teilanmeldung aus: 04724014.8
(71) Anmelder: Morphochem Aktiengesellschaft für kombinatorische Chemie, 81379 München (DE)
(72) Erfinder: Hubschwerlen, Christian, 68480 Durmenach (FR); Surivet, Jean Philippe, 68300 Saint-Louis (FR); Zumbrunn, Cornelia, 4058 Basel (CH)
(74) Vertreter: Forstmeyer, Dietmar

(57) **Zusammenfassung**

Die vorliegende Anmeldung beschreibt neuartige antibakterielle Verbindungen der Formel (I). Diese Verbindungen sind unter anderem als Inhibitoren von Topoisomerase IV (Topo IV) sowie von DNA Gyrase von Interesse.

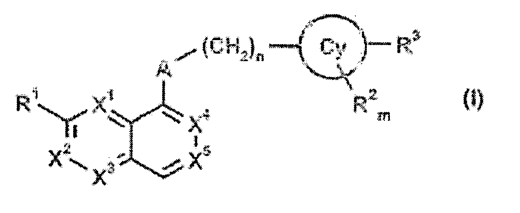

## Beschreibung

In vielen Ländern der Welt hat die Resistenz gegenüber den derzeit gebräuchlichen Antibiotika in den letzten Jahren beträchtlich zugenommen und zum Teil bedrohliche Ausmasse angenommen. Das Hauptproblem dabei ist, dass diese Erreger nicht nur eine, sondern in der Regel mehrfache Resistenzen tragen. Dies gilt insbesondere für einige Gram-positive Erregergruppen, wie Staphylokokken, Pneumokokken und Enterokokken (S. Ewig et al.; Antibiotika-Resistenz bei Erregern ambulant erworbener Atemwegsinfektionen; Chemother. J. 2002, 11, 12-26; F. Tenover; Development and spread of bacterial resistance to antimicrobial agents: an overview; Clin. Infect. Dis. 2001 Sep 15, 33 Suppl. 3, 108-115)

Eine lange befürchtete Entwicklung ist kürzlich eingetreten: In den USA wurde der erste Stamm von *Staphylococcus* aureus beschrieben, welcher nicht nur Methicillin-resistent, sondern auch gegen Vancomycin hoch-resistent ist (Centers for Disease Control and Prevention; Staphylococcus aureus resistant to vancomycin - United States, 2002; MMWR 2002, 51, 565-567).

Neben hygienischen Massnahmen in Krankenhäusern sind daher auch verstärkt Anstrengungen erforderlich, neue Antibiotika zu finden, die möglichst eine neue Struktur und einen neuen Wirkungsmechanismus besitzen, um gegen diese Problemkeime wirksam zu sein.

Die vorliegende Anmeldung beschreibt neuartige Verbindungen mit antibakterieller Aktivität. Diese Verbindungen sind unter anderem als Inhibitoren von Topoisomerase IV (Topo IV) sowie von DNA Gyrase von Interesse.

Die vorliegende Erfindung betrifft Verbindungen der allgemeinen Formel (I): 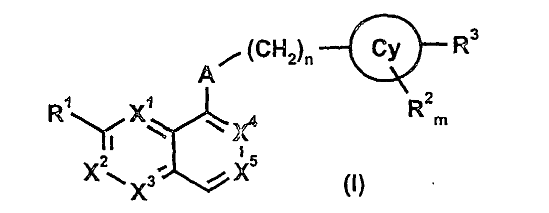 wobei
A ein Sauerstoff- oder ein Schwefelatom, eine NH, eine Alkylen-, eine Alkenylen-, eine Alkinylen- oder eine Heteroalkylengruppe ist,
X¹, X², X³, X⁴ und X⁵ unabhängig voneinander Stickstoffatome oder Gruppen der Formel CH oder CR⁴ sind,
Cy eine Cycloalkylen-, eine Heterocycloalkylen-, eine Arylen- oder eine Heteroarylengruppe ist.
R¹ ein Wasserstoffatom, ein Halogenatom, eine Hydroxy-, eine Amino-, eine Thiol-, eine Alkyl-, eine Heteroalkyl-, eine Alkyloxy-, eine Heteroalkyloxy-, eine Cyloalkyl-, eine Heterocycloalkyl-, eine Alkylcycloalkyl-, eine Heteroalkylcycloalkyl-, eine Cycloalkyloxy-, eine Alkylcycloalkyloxy-, eine Heterocycloalkyloxy oder eine Heteroalkylcycloalkyloxygruppe ist,
die Reste R² unabhängig voneinander ein Halogenatom, eine Hydroxy-, Amino-, Nitro- oder Thiolgruppe, ein Alkyl-, Alkenyl-, Alkinyl-, Heteroalkyl-, Aryl-, Heteroaryl-, Cycloalkyl-, Alkylcycloalkyl-, Heteroalkylcycloalkyl-, Heterocycloalkyl-, Aralkyl- oder ein Heteroaralkylrest sind, oder zwei der Reste R² zusammen Teil eines Aryl-, Heteroaryl-, Cycloalkyl-, Heterocycloalkyl-, Alkylcycloalkyl-, Heteroalkylcycloalkyl-, Aralkyl- oder ein Heteroaralkylrings sind,
R³ ein Alkyl-, Alkenyl-, Alkinyl-, Heteroalkyl-, Aryl-, Heteroaryl-, Cycloalkyl-, Alkylcycloalkyl-, Heteroalkylcycloalkyl-, Heterocycloalkyl-, Aralkyl- oder ein Heteroaralkylrest ist,
R⁴ ein Halogenatom, eine Hydroxy-, Alkyl-, Alkenyl-, Alkinyl- oder eine Heteroalkylgruppe ist,
n gleich 0, 1 oder 2 ist und
m gleich 0, 1, oder 2 ist,
oder ein pharmakologisch akzeptables Salz, Solvat, Hydrat oder eine pharmakologisch akzeptable Formulierung derselben.

Der Ausdruck Alkyl bezieht sich auf eine gesättigte, geradkettige oder verzweigte Kohlenwasserstoffgruppe, die 1 bis 20 Kohlenstoffatome, vorzugsweise 1 bis 12 Kohlenstoffatome, besonders bevorzugt 1 bis 6 Kohlenstoffatome aufweist, z.B. die Methyl-, Ethyl-, Propyl-, Isopropyl-, n-Butyl-, Isobutyl-, tert-Butyl, n-Pentyl-, n-Hexyl-, 2,2-Dimethylbutyl- oder n-Octyl-Gruppe.

Die Ausdrücke Alkenyl und Alkinyl beziehen sich auf zumindest teilweise ungesättigte, geradkettige oder verzweigte Kohlenwasserstoffgruppen, die 2 bis 20 Kohlenstoffatome, vorzugsweise 2 bis 12 Kohlenstoffatome, besonders bevorzugt 2 bis 6 Kohlenstoffatome aufweisen, z. B. die Ethenyl-, Allyl-, Acetylenyl-, Propargyl-, Isoprenyl- oder Hex-2-enyl-Gruppe. Bevorzugt weisen Alkenylgruppen eine oder zwei (besonders bevorzugt eine) Doppelbindungen bzw. Alkinylgruppen eine oder zwei (besonders bevorzugt eine) Dreifachbindungen auf.

Des weiteren beziehen sich die Begriffe Alkyl, Alkenyl und Alkinyl auf Gruppen, bei der ein oder mehrere Wasserstoffatome durch ein Halogenatom (bevorzugt F oder Cl) ersetzt sind wie z. B. die 2,2,2-Trichlorethyl-, oder die Trifluormethylgruppe.

Der Ausdruck Heteroalkyl bezieht sich auf eine Alkyl-, eine Alkenyl- oder eine Alkinyl-Gruppe, in der ein oder mehrere (bevorzugt 1, 2 oder 3) Kohlenstoffatome durch ein Sauerstoff-, Stickstoff-, Phosphor-, Bor-, Selen-, Silizium- oder Schwefelatom ersetzt sind (bevorzugt Sauerstoff, Schwefel oder Stickstoff). Der Ausdruck Heteroalkyl bezieht sich des weiteren auf eine Carbonsäure oder eine von einer Carbonsäure abgeleitete Gruppe wie z. B. Acyl, Acylalkyl, Alkoxycarbonyl, Acyloxy, Acyloxyalkyl, Carboxyalkylamid oder Alkoxycarbonyloxy.

Beispiele für Heteroalkylgruppen sind Gruppen der Formeln R^{a}-O-Y^{a}-, R^{a}-S-Y^{a}-, R^{a}-N(R^{b})-Y^{a}-, R^{a}-CO-Y^{a}-, R^{a}-O-CO-Y^{a}-, R^{a}-CO-O-Y^{a}-, R^{a}-CO-N (R^{b})-Y^{a}-, R^{a}-N (R^{b}) -CO-Y^{a}-, R^{a}-O-CO-N(R^{b})-Y^{a}-, R^{a}-N(R^{b})-CO-O-Y^{a}-, R^{a}-N(R^{b})-CO-N(R^{c})-Y^{a}-, R^{a}-O-CO-O-Y^{a}- R^{a}-N (R^{b}) -C (=NR^{d}) ⁻N(R^{c})-Y^{a}-, R^{a}-CS-Y^{a}-, R-O-CS-Y^{a}-, R^{a}-CS-O-Y^{a}-, R^{a}-CS-N(R^{b})-Y^{a}-, R^{a}-N(R^{b}) -CS-Y^{a}-, R^{a}-O-CS-N (R^{b}) -Y^{a}-, R^{a}-N (R^{b})-CS-O-Y^{a}-, R^{a}-N(R^{b})-CS-N(R^{c})-Y^{a}-, R^{a}-O-CS-O-Y^{a}-, R^{a}-S-CO-Y^{a}-, R^{a}-CO-S-Y^{a}-, R^{a}-S-CO-N(R^{b})-Y^{a}-, R^{a}-N(R^{b})-CO-S-Y^{a}-, R^{a}-S-CO-O-Y^{a}-, R^{a}-O-CO-S-Y^{a}-, R^{a}-S-CO-S-Y^{a}-, R^{a}-S-CS-Y^{a}-, R^{a}-CS-S-Y^{a}-, R^{a}-S-CS-N(R^{b})-Y^{a}-, R^{a}-N(R^{b})-CS-S-Y^{a}-, R^{a}-S-CS-O-Y^{a}-, R^{a}-O-CS-S-Y^{a}-, wobei R^{a} ein Wasserstoffatom, eine C₁-C₆-Alkyl-, eine C₂-C₆-Alkenyl- oder eine C₂-C₆-Alkinylgruppe; R^{b} ein Wasserstoffatom, eine C₁-C₆-Alkyl-, eine C₂-C₆-Alkenyl- oder eine C₂-C₆-Alkinylgruppe; R^{c} ein Wasserstoffatom, eine C₁-C₆-Alkyl-, eine C₂-C₆-Alkenyl- oder eine C₂-C₆-Alkinylgruppe; R^{d} ein Wasserstoffatom, eine C₁-C₆-Alkyl-, eine C₂-C₆-Alkenyl- oder eine C₂-C₆-Alkinylgruppe und Y^{a} eine direkte Bindung, eine C₁-C₆-Alkylen-, eine C₂-C₆-Alkenylen- oder eine C₂-C₆-Alkinylengruppe ist, wobei jede Heteroalkylgruppe mindestens ein Kohlenstoffatom enthält und ein oder mehrere Wasserstoffatome durch Fluor- oder Chloratome ersetzt sein können. Konkrete Beispiele für Heteroalkylgruppen sind Methoxy, Trifluormethoxy, Ethoxy, n-Propyloxy, iso-Propyloxy, tert-Butyloxy, Methoxymethyl, Ethoxymethyl, Methoxyethyl, Methylamino, Ethylamino, Dimethylamino, Diethylamino, iso-Propylethylamino, Methyl-aminomethyl, Ethylaminomethyl, Di-isoPropylaminoethyl, Enolether, Dimethylaminomethyl, Dimethylaminoethyl, Acetyl, Propionyl, Butyryloxy, Acetyloxy, Methoxycarbonyl, Ethoxycarbonyl, N-Ethyl-N-Methylcarbamoyl oder N-Methylcarbamoyl. Weitere Beispiele für Heteroalkylgruppen sind Nitril-, Isonitril, Cyanat-, Thiocyanat-, Isocyanat-, Isothiocyanat und Alkylnitrilgruppen.

Der Ausdruck Cycloalkyl bezieht sich auf eine gesättigte oder teilweise ungesättigte (z. B. Cycloalkenyl) cyclische Gruppe, die einen oder mehrere Ringe (bevorzugt 1 oder 2) aufweist, die ein Gerüst bilden, welches 3 bis 14 Kohlenstoffatome, vorzugsweise 3 bis 10 (insbesondere 3, 4, 5, 6 oder 7) Kohlenstoffatome enthält. Der Ausdruck Cycloalkyl bezieht sich weiterhin auf Gruppen, bei denen ein oder mehrere Wasserstoffatome durch Fluor-, Chlor-, Brom- oder Jodatome oder OH, =O, SH, =S, NH₂, =NH oder NO₂-Gruppen ersetzt sind also z. B. cyclische Ketone wie z. B. Cyclohexanon, 2-Cyclohexenon oder Cyclopentanon. Weitere konkrete Beispiele für Cycloalkylgruppen sind die Cyclopropyl-, Cyclobutyl-, Cyclopentyl-, Spiro[4,5]-decanyl-, Norborny-, Cyclohexyl-, Cyclopentenyl-, Cyclohexadienyl-, Decalinyl-, Bicyclo[4.3.0]nonyl-, Tetralin-, Cyclopentylcyclohexyl-, Fluorcyclohexyl- oder die Cyclohex-2-enyl-Gruppe.

Der Ausdruck Heterocycloalkyl bezieht sich auf eine Cycloalkylgruppe wie oben definiert, in der ein oder mehrere (bevorzugt 1, 2 oder 3) Ring-Kohlenstoffatome durch ein Sauerstoff-, Stickstoff-, Silizium-, Selen-, Phosphor- oder Schwefelatom (bevorzugt Sauerstoff, Schwefel oder Stickstoff) ersetzt sind. Bevorzugt besitzt eine Heterocycloalkylgruppe 1 oder 2 Ringe mit 3 bis 10 (insbesondere 3, 4, 5, 6 oder 7) Ringatomen. Der Ausdruck Heterocycloalkyl bezieht sich weiterhin auf Gruppen, bei denen ein oder mehrere Wasserstoffatome durch Fluor-, Chlor-, Brom- oder Jodatome oder OH, =O, SH, =S, NH₂, =NH oder NO₂-Gruppen ersetzt sind. Beispiele sind die Piperidyl-, Piperazinyl-, Morpholinyl-, Urotropinyl-, Pyrrolidinyl-, Tetrahydrothiophenyl-, Tetrahydropyranyl-, Tetrahydrofuryl- oder 2-Pyrazolinyl-Gruppe sowie Lactame, Lactone, cyclische Imide und cyclische Anhydride.

Der Ausdruck Alkylcycloalkyl bezieht sich auf Gruppen, die entsprechend den obigen Definitionen sowohl Cycloalkyl- wie auch Alkyl-, Alkenyl- oder Alkinylgruppen enthalten, z. B. Alkylcycloalkyl-, Cycloalkylalkyl-, Alkylcycloalkenyl-, Alkenylcycloalkyl- und Alkinylcycloalkylgruppen. Bevor-zugt enthält eine Alkylcycloalkylgruppe eine Cycloalkyl-gruppe, die einen oder zwei Ringsysteme aufweist, die ein Gerüst bilden, welches 3 bis 10 (insbesondere 3, 4, 5, 6 oder 7) Kohlenstoffatome enthält und eine oder zwei Alkyl-, Alkenyl- oder Alkinylgruppen mit 1 oder 2 bis 6 Kohlenstoffatomen.

Der Ausdruck Heteroalkylcycloalkyl bezieht sich auf Alkylcycloalkylgruppen, wie oben definiert, in der ein oder mehrere (bevorzugt 1, 2 oder 3) Kohlenstoffatome durch ein Sauerstoff-, Stickstoff-, Silizium-, Selen-, Phosphor- oder Schwefelatom (bevorzugt Sauerstoff, Schwefel oder Stickstoff) ersetzt sind. Bevorzugt besitzt eine Heteroakylcycloalkylgruppe 1 oder 2 Ringsysteme mit 3 bis 10 (insbesondere 3, 4, 5, 6 oder 7) Ringatomen und eine oder zwei Alkyl, Alkenyl, Alkinyl oder Heteroalkylgruppen mit 1 oder 2 bis 6 Kohlenstoffatomen. Beispiele derartiger Gruppen sind Alkylheterocycloalkyl, Alkylheterocycloalkenyl, Alkenylheterocycloalkyl, Alkinylheterocycloalkyl, Heteroalkylcycloalkyl, Heteroalkylheterocycloalkyl und Heteroalkylheterocylcloalkenyl, wobei die cyclischen Gruppen gesättigt oder einfach, zweifach oder dreifach ungesättigt sind.

Der Ausdruck Aryl bzw. Ar bezieht sich auf eine aromatische Gruppe, die einen oder mehrere Ringe hat, und durch ein Gerüst gebildet wird, das 6 bis 14 Kohlenstoffatome, vorzugsweise 6 bis 10 (insbesondere 6) Kohlenstoffatome enthält. Der Ausdruck Aryl (bzw. Ar) bezieht sich weiterhin auf Gruppen, bei denen ein oder mehrere Wasserstoffatome durch Fluor-, Chlor-, Brom- oder Jodatome oder OH, SH, NH₂, oder NO₂-Gruppen ersetzt sind. Beispiele sind die Phenyl-, Naphthyl-, Biphenyl-, 2-Fluorphenyl, Anilinyl-, 3-Nitrophenyl oder 4-Hydroxyphenyl-Gruppe.

Der Ausdruck Heteroaryl bezieht sich auf eine aromatische Gruppe, die einen oder mehrere Ringe hat, und durch ein Gerüst gebildet wird, das 5 bis 14 Ringatome, vorzugsweise 5 bis 10 (insbesondere 5 oder 6) Ringatome enthält und ein oder mehrere (bevorzugt 1, 2, 3 oder 4) Sauerstoff-, Stickstoff-, Phosphor- oder Schwefel-Ringatome (bevorzugt O, S oder N) enthält. Der Ausdruck Heteroaryl bezieht sich weiterhin auf Gruppen, bei denen ein oder mehrere Wasserstoffatome durch Fluor-, Chlor-, Brom- oder Jodatome oder OH, SH, NH₂, oder NO₂-Gruppen ersetzt sind. Beispiele sind 4-Pyridyl-, 2-Imidazolyl-, 3-Phenylpyrrolyl-, Thiazolyl-, Oxazolyl-, Triazolyl-, Tetrazolyl-, Isoxazolyl-, Indazolyl-, Indolyl-, Benzimidazolyl-, Pyridazinyl-, Chinolinyl-, Purinyl-, Carbazolyl-, Acridinyl-, Pyrimidyl-, 2,3'-Bifuryl-, 3-Pyrazolyl- und Isochinolinyl-Gruppen.

Der Ausdruck Aralkyl bezieht sich auf Gruppen, die entsprechend den obigen Definitionen sowohl Aryl- wie auch Alkyl-, Alkenyl-, Alkinyl- und/oder Cycloalkylgruppen enthalten, wie z. B. Arylalkyl-, Arylalkenyl-, Arylalkinyl-, Arylcycloalkyl-, Arylcycloalkenyl-, Alkylarylcycloalkyl- und Alkylarylcycloalkenylgruppen. Konkrete Beispiele für Aralkyle sind Toluol, Xylol, Mesitylen, Styrol, Benzylchlorid, o-Fluortoluol, 1H-Inden, Tetralin, Dihydronaphthaline, Indanon, Phenylcyclopentyl, Cumol, Cyclohexylphenyl, Fluoren und Indan. Bevorzugt enthält eine Aralkylgruppe ein oder zwei aromatische Ringsysteme (1 oder 2 Ringe) mit 6 bis 10 Kohlenstoffatomen und ein oder zwei Alkyl-, Alkenyl- und/oder Alkinylgruppen mit 1 oder 2 bis 6 Kohlenstoffatomen und/oder eine Cycloalkylgruppe mit 5 oder 6 Ringkohlenstoffatomen.

Der Ausdruck Heteroaralkyl bezieht sich auf eine Aralkylgruppe wie oben definiert, in der ein oder mehrere (bevorzugt 1, 2, 3 oder 4) Kohlenstoffatome durch ein Sauerstoff-, Stickstoff-, Silizium-, Selen-, Phosphor-, Bor- oder Schwefelatom (bevorzugt Sauerstoff, Schwefel oder Stickstoff) ersetzt sind, d. h. auf Gruppen, die entsprechend den obigen Definitionen sowohl Aryl- bzw. Heteroaryl- wie auch Alkyl-, Alkenyl-, Alkinyl- und/oder Heteroalkyl- und/oder Cycloalkyl- und/oder Heterocycloalkylgruppen enthalten. Bevorzugt enthält eine Heteroaralkylgruppe ein oder zwei aromatische Ringsysteme (1 oder 2 Ringe) mit 5 oder 6 bis 10 Kohlenstoffatomen und ein oder zwei Alkyl-, Alkenyl- und/oder Alkinylgruppen mit 1 oder 2 bis 6 Kohlenstoffatomen und/oder eine Cycloalkylgruppe mit 5 oder 6 Ringkohlenstoffatomen, wobei 1, 2, 3 oder 4 dieser Kohlenstoffatome durch Sauerstoff-, Schwefel- oder Stickstoffatome ersetzt sind.

Beispiele sind Arylheteroalkyl-, Arylheterocycloalkyl-, Arylheterocycloalkenyl-, Arylalkylheterocycloalkyl-, Arylalkenylheterocycloalkyl-, Arylalkinylheterocycloalkyl-, Arylalkylheterocycloalkenyl-, Heteroarylalkyl-, Heteroarylalkenyl-, Heteroarylalkinyl-, Heteroarylheteroalkyl-, Heteroarylcycloalkyl-, Heteroarylcycloalkenyl-, Heteroarylheterocycloalkyl-, Heteroarylheterocycloalkenyl-, Heteroarylalkylcycloalkyl-, Heteroarylalkylheterocycloalkenyl-, Heteroarylheteroalkylcycloalkyl-, Heteroarylheteroalkylcycloalkenyl- und Heteroarylheteroalkylheterocycloalkyl-Gruppen, wobei die cyclischen Gruppen gesättigt oder einfach, zweifach oder dreifach ungesättigt sind. Konkrete Beispiele sind die Tetrahydroisochinolinyl-, Benzoyl-, 2- oder 3-Ethylindolyl-, 4-Methylpyridino-, 2-, 3- oder 4-Methoxyphenyl-, 4-Ethoxyphenyl-, 2-, 3- oder 4-Carboxyphenylalkylgruppe.

Die Ausdrücke Cycloalkyl, Heterocycloalkyl, Alkylcycloalkyl, Heteroalkylcycloalkyl, Aryl, Heteroaryl, Aralkyl und Heteroaralkyl beziehen sich auch auf Gruppen, in denen ein oder mehrere Wasserstoffatome solcher Gruppen durch Fluor-, Chlor-, Brom- oder Jodatome oder OH, =O, SH, =S, NH₂, =NH oder NO₂-Gruppen ersetzt sind.

Der Ausdruch "gegebenenfalls substituiert" bezieht sich auf Gruppen, in denen ein oder mehrere Wasserstoffatome durch Fluor-, Chlor-, Brom- oder Jodatome oder OH, =O, SH, =S, NH₂, =NH oder NO₂-Gruppen ersetzt sind. Dieser Ausdruck bezieht sich weiterhin auf Gruppen, die mit unsubstituierten C₁-C₆ Alkyl-, C₂-C₆ Alkenyl-, C₂-C₆ Alkinyl-, C₁-C₆ Heteroalkyl-, C₃-C₁₀ Cycloalkyl-, C₂-C₉ Heterocycloalkyl-, C₆-C₁₀ Aryl-, C₁-C₉ Heteroaryl-, C₇-C₁₂ Aralkyl- oder C₂-C₁₁ Heteroaralkyl-Gruppen substituiert sind.

Verbindungen der Formel (I) können aufgrund ihrer Substitution ein oder mehrere Chiralitätszentren enthalten. Die vorliegende Erfindung umfasst daher sowohl alle reinen Enantiomere und alle reinen Diastereomere, als auch deren Gemische in jedem Mischungsverhältnis. Des weiteren sind von der vorliegenden Erfindung auch alle cis/trans-Isomeren der Verbindungen der allgemeinen Formel (I) sowie Gemische davon umfasst. Des weiteren sind von der vorliegenden Erfindung alle tautomeren Formen der Verbindungen der Formel (I) umfasst.

Bevorzugt sind Verbindungen der Formel (I), wobei A ein Sauerstoff- oder ein Schwefelatom oder eine Gruppe der Formel CH₂, CH₂CH₂, CH₂N(C₁-C₄-Alkyl), N(C₁-C₄-Alkyl)CH₂, CH₂O, OCH₂, CH₂S, SCH₂, CH₂CH(OH), CH (OH), CH(OH)CH₂, NHCO, CONH, C(=O) CH₂ oder CH₂C(=O) ist.

Weiter bevorzugt sind Verbindungen der Formel (I), wobei drei, vier oder fünf der Gruppen X¹, X², X³, X⁴ und X⁵, CH-Gruppen sind.

Wiederum bevorzugt ist R¹ eine C₁-C₄-Alkyloxy- oder eine C₁-C₄-Heteroalkyloxygruppe, wobei ein oder mehrere Wasserstoffatome dieser Gruppen durch Fluoratome ersetzt sein können.

Besonders bevorzugt ist R¹ eine Methoxygruppe.

Wiederum bevorzugt ist R² eine Hydroxy-, eine C₁-C₄-Alkyl-, C₁-C₄-Heteroalkyl- oder eine C₆-C₁₂-Heteroaralkylgruppe.

Weiter bevorzugt ist R³ eine Heteroalkylcycloalkyl- oder eine Heteroaralkylgruppe.

Besonders bevorzugt ist R³ eine Gruppe der Formel -B-Y, wobei B eine Alkylen- (insbesondere eine C₁-C₄-Alkylengruppe), Alkenylen-, Alkinylen- oder eine Heteroalkylengruppe (insbesondere eine C₁-C₄-Heteroalkylengruppe) ist und Y eine Aryl-, Heteroaryl-, Aralkyl-, Heteroaralkyl-, Cycloalkyl-, Heterocycloalkyl-, Alkylcycloalkyl- oder eine Heteroalkylcycloalkylgruppe (insbesondere eine Heterocycloalkyl- oder eine Arylheterocycloalkylgruppe) ist.

Wiederum bevorzugt weist Y eine der folgenden Strukturen auf 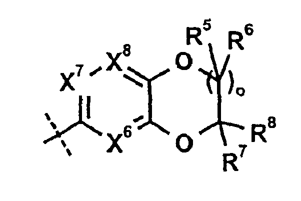 oder 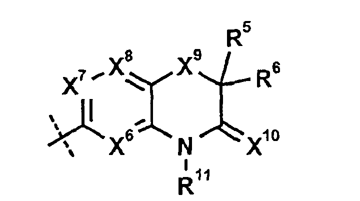 wobei X⁶, X⁷ und X⁸ unabhängig voneinander Stickstoffatome oder Gruppen der Formel CR⁹ sind, X⁹ und X¹⁰ unabhängig voneinander Sauerstoff- oder Schwefelatome oder Gruppen der Formel NR¹⁰ sind, o gleich 0, 1 oder 2 ist, R⁵, R⁶, R⁷, R⁸ und R⁹ unabhängig voneinander Wasserstoffatome, Halogenatome, Hydroxy-, Alkyl-, Alkenyl-, Alkinyl- oder Heteroalkylgruppen sind und R¹⁰ und R¹¹ unabhängig voneinander Wasserstoffatome, Alkyl-, Alkenyl-, Alkinyl- oder Heteroalkylgruppen sind.

Besonders bevorzugt weist Y eine der folgenden Strukturen auf: 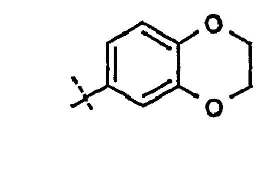 oder

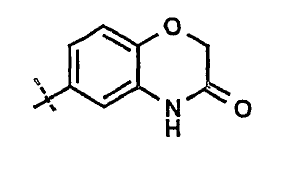

Weiter bevorzugt hat der Linker -A-(CH₂)- eine Kettenlänge von 2 oder 3 Atomen.

Des weiteren bevorzugt ist R⁴ ein Fluor- oder ein Chloratom oder eine C₁-C₄-Alkyloxy-, oder eine C₃-C₆-Dialkylaminomethylgruppe, wobei ein oder mehrere Wasserstoffatome dieser Gruppen durch Fluoratome ersetzt sein können.

Wiederum bevorzugt ist Cy eine Cycloalkylen- oder eine Heterocycloalkylengruppe mit ein oder zwei Ringen und 4, 5, 6, 7, 8, 9 oder 10 Ringatomen.

Besonders bevorzugt ist Cy eine Gruppe der Formeln 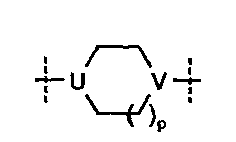 oder 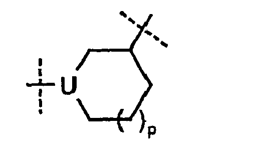 wobei U ein Stickstoffatom oder eine Gruppe der Formel CH oder COH ist und V ein Stickstoffatom oder eine CH-Gruppe ist und p gleich 0 oder 1 ist. An diese Gruppe können die Substituenten jeweils sowohl equatorial als auch axial gebunden sein.

Die therapeutische Verwendung der Verbindungen der Formel (I), ihrer pharmakologisch akzeptablen Salze bzw. Solvate und Hydrate sowie Formulierungen und pharmazeutischen Zusammensetzungen liegt ebenfalls im Rahmen der vorliegenden Erfindung.

Die pharmazeutischen Zusammensetzungen gemäß der vorliegenden Erfindung enthalten mindestens eine Verbindung der Formel (I) als Wirkstoff und fakultativ Trägerstoffe und/oder Adjuvantien.

Beispiele für pharmakologisch akzeptable Salze der Verbindungen der Formel (I) sind Salze von physiologisch akzeptablen Mineralsäuren wie Salzsäure, Schwefelsäure und Phosphorsäure oder Salze von organischen Säuren wie Methansulfonsäure, p-Toluolsulfonsäure, Milchsäure, Essigsäure, Trifluoressigsäure, Zitronensäure, Bernsteinsäure, Fumarsäure, Maleinsäure und Salicylsäure. Weitere Beispiele für pharmakologisch akzeptable Salze der Verbindungen der Formel (I) sind Alkali- oder Erdalkalisalze wie z. B. Natrium, Kalium, Lithium, Calcium oder Magnesium Salze, Ammoniumsalze oder Salze von organischen Basen wie z. B. Methylamin, Dimethylamin, Triethylamin, Piperidin, Ethylendiamin, Lysin, Cholinhydroxid, Meglumin, Morpholin oder Arginin Salze. Verbindungen der Formel (I) können solvatisiert, insbesondere hydratisiert sein. Die Hydratisierung kann z.B. während des Herstellungsverfahrens oder als Folge der hygroskopischen Natur der anfänglich wasserfreien Verbindungen der Formel (I) auftreten. Wenn die Verbindungen der Formel (I) asymmetrische C-Atome enthalten, können sie entweder als achirale Verbindungen, Diastereomeren-Gemische, Gemische von Enantiomeren oder als optisch reine Verbindungen vorliegen.

Die Pro-Drugs, die ebenfalls Gegenstand der vorliegenden Erfindung sind, bestehen aus einer Verbindung der Formel (I) und mindestens einer pharmakologisch akzeptablen Schutzgruppe, die unter physiologischen Bedingungen abgespalten wird, z.B. einer Alkoxy-, Aralkyloxy-, Acyl- oder Acyloxy-Gruppe, wie z.B. einer Ethoxy-, Benzyloxy-, Acetyl- oder Acetyloxy-Gruppe.

Auch die Verwendung dieser Wirkstoffe zur Herstellung von Arzneimitteln ist Gegenstand der vorliegenden Erfindung. Im allgemeinen werden Verbindungen der Formel (I) unter Anwendung der bekannten und akzeptablen Modi, entweder einzeln oder in Kombination mit einem beliebigen anderen therapeutischen Mittel verabreicht. Solche therapeutisch nützlichen Mittel können auf einem der folgenden Wege verabreicht werden: oral, z.B. als Dragees, überzogene Tabletten, Pillen, Halbfeststoffe, weiche oder harte Kapseln, Lösungen, Emulsionen oder Suspensionen; parenteral, z.B. als injizierbare Lösung; rektal als Suppositorien; durch Inhalation, z.B. als Pulverformulierung oder Spray, transdermal oder intranasal. Zur Herstellung solcher Tabletten, Pillen, Halbfeststoffe, überzogenen Tabletten, Dragees und harten Gelatinekapseln kann das therapeutisch verwendbare Produkt mit pharmakologisch inerten, anorganischen oder organischen Arzneimittelträgersubstanzen vermischt werden, z.B. mit Lactose, Sucrose, Glucose, Gelatine, Malz, Silicagel, Stärke oder Derivaten derselben, Talkum, Stearinsäure oder ihren Salzen, Trockenmagermilch und dgl. Zur Herstellung von weichen Kapseln kann man Arzneimittelträgerstoffe wie z.B. pflanzliche Öle, Petroleum, tierische oder synthetische Öle, Wachs, Fett, Polyole einsetzen. Zur Herstellung von flüssigen Lösungen und Sirups kann man Arzneimittelträgerstoffe wie z.B. Wasser, Alkohole, wäßrige Salzlösung, wäßrige Dextrose, Polyole, Glycerin, pflanzliche Öle, Petroleum, tierische oder synthetische Öle verwenden. Für Suppositorien kann man Arzneimittelträgerstoffe wie z.B. pflanzliche Öle, Petroleum, tierische oder synthetische Öle, Wachs, Fett und Polyole verwenden. Für Aerosol-Formulierungen kann man komprimierte Gase, die für diesen Zweck geeignet sind, wie z.B. Sauerstoff, Stickstoff und Kohlendioxid einsetzen. Die pharmazeutisch verwendbaren Mittel können auch Zusatzstoffe zur Konservierung, Stabilisierung, Emulgatoren, Süßstoffe, Aromastoffe, Salze zur Veränderung des osmotischen Drucks, Puffer, Umhüllungszusatzstoffe und Antioxidantien enthalten.

Kombinationen mit anderen therapeutischen Mitteln können andere antimikrobielle und antifungale Wirkstoffe beinhalten.

Zur Vorbeugung und/oder Behandlung der oben beschriebenen Erkrankungen kann die Dosis der erfindungsgemäßen biologisch aktiven Verbindung innerhalb breiter Grenzen variieren und kann auf den individuellen Bedarf eingestellt werden. Im allgemeinen ist eine Dosis von 10 mg bis 4000 mg pro Tag geeignet, wobei eine bevorzugte Dosis 50 bis 3000 mg pro Tag ist. In geeigneten Fällen kann die Dosis auch unter oder über den oben angegebenen Werten liegen. Die tägliche Dosis kann als einfache Gabe oder in mehrfachen Gaben verabreicht werden. Eine typische Einzeldosis beinhaltet etwa 50 mg, 100 mg, 250 mg, 500 mg, 1 g oder 2 g des Wirkstoffs.

### BEISPIELE

### Beispiel 1: (R,S)-6-{1-Hydroxy-2-[4-(7-methoxynaphthalen-1-yloxymethyl)-piperidin-1-yl]-ethyl}-4H-benzo[1,4]oxazin-3-on

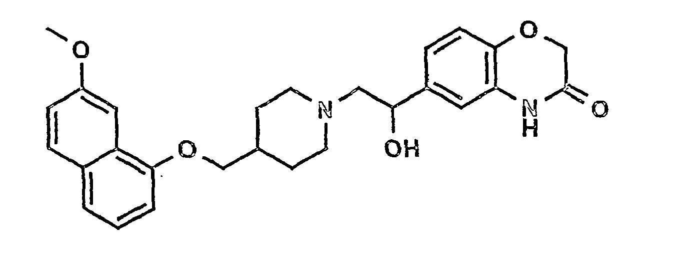

### Synthese von 4-(7-Methoxy-naphthalen-1-yloxymethyl)-piperidin-1-carbonsäure tert-butyl ester

Zu einer Lösung von Triphenylphosphin (1.14 g; 4.3 mmol) in THF (5 ml) wurde tropfenweise Diethyazodicarboxylat (755mg; 4.3 mmol) zugetropft. 4-Hydroxymethyl-piperidine-1-carbonsäure tert-butyl ester (850 mg, 3.95 mmol) wurde zugegeben, gefolgt von 7-methoxy-1-naphthol (synthetisiert analog Aust. J. Chem. 1993, 46, 731) (668 mg; 3.95 mmol). Die gelbe Lösung wurde über Nacht bei Raumtemperatur gerührt, eingeengt und mittels Säulenchromatographie an Silica Gel (hex/EtOAc 4:1) gereinigt. Man erhielt 1.11g (76%) eines farblosen Oels.
MS (ESI⁺) : 372.3 [M+H⁺]

### Synthese von 4-(7-Methoxy-naphthalen-2-yloxymethyl)-piperidin

Eine Lösung von 4-(7-Methoxy-naphthalen-1-yloxymethyl)-piperidine-1-carbonsäure tert-butyl ester (1,.11 g) in Dichlormethan (10 ml) wurde bei Raumtemperatur unter Argon mit Trifluoressigsäure (2 ml) versetzt und während zwei Stunden gerührt. Das Reaktionsgemisch wurde am Rotationsverdampfer eingeengt, in Dichlormethan aufgenommen und mit conc. Ammoniaklösung gewaschen. Die organischen Phasen wurden über MgSO₄ getrocknet und eingeengt.

### Synthese von 6-{2-[4-(7-Methoxy-naphthalen-1-yloxymethyl)-piperidin-1-yl]-acetyl}-4H-benzo[1,4]oxazin-3-on

Ein Gemisch aus 4-(7-Methoxy-naphthalen-1-yloxymethyl)-piperidin (271 mg, 1 mmol) und 6-(2-Chloro-acetyl)-4H-benzo[1,4]oxazin-3-on (225 mg, 1 mmol) in THF (5 ml) wurde mit Triethylamin (1 ml) versetzt und während 2h auf 50°C erhitzt. Das Reaktionsgemisch wurde auf Wasser gegossen und mit EtOAc extrahiert. Organische Phasen mit NH₄Cl Lösung gewaschen, über MgSO₄ getrocknet und eingeengt. Der kristalline Rückstand wurde mit MeOH und EtOAc verrührt und abgenutscht. Man erhielt 250 mg (52%) reines Produkt.
MS (ESI⁺) 461 [M+H⁺]

### Synthese von (R,S)-6-{1-Hydroxy-2-[4-(7-methoxynaphthalen-1-yloxymethyl)-piperidin-1-yl]-ethyl}-4H-benzo[1,4]oxazin-3-on

Eine Lösung von 6-{2-[4-(7-Methoxy-naphthalen-1-yloxymethyl)-piperidin-1-yl]-acetyl}-4H-benzo[1,4]oxazin-3-on (150 mg) in EtOH (2 ml) wurde mit NaBH₄ (1 eq) versetzt und während 2h bei rt gerührt. Das Reaktionsgemisch wurde eingeengt, in Wasser aufgenommen und die weissen Kristalle abgenutscht und unter Hochvakuum getrocknet. Man erhielt 140 mg reines Produkt.
MS (ESI⁺) 463.5 [M+H⁺]

### Beipiel 2: (R,S)-1-(2,3-Dihydro-benzo[1,4]dioxin-6-yl)-2-[4-(7-methoxy-naphthalen-1-yloxymethyl)-piperidin-1-yl]-ethanon

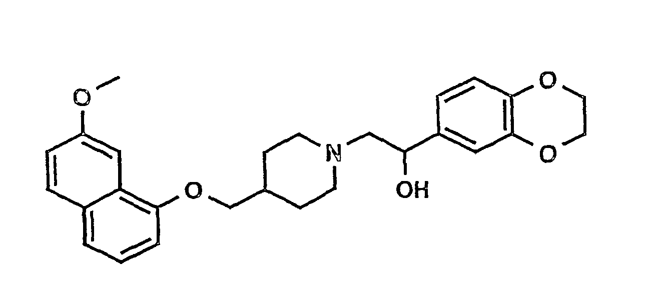

### Synthese von 6-Oxiranyl-2,3-dihydro-benzo[1,4]dioxin

In einem 50ml Rundkolben wurde 2,3-Dihydrobenzo[1,4]dioxine-6-carbaldehyde (1g, 6.09 mmol) in Acetonitril (15 ml) gelöst, Trimethylsulfoniumjodid (1.28g, 6.28 mmol) und KOH (2.4 g) und einige Tropfen Wasser dazugegeben und 1.5h bei 60°C rühren gelassen. Das Reaktionsgemisch wurde am Rotationsverdampfer eingeengt. Der Rückstand wurde mit Wasser aufgenommen und mit EtOAc extrahiert. Die organische Phase wurde über Na₂SO₄ getrocknet, filtriert und eingeengt. Der Rückstand wurde mit Hex1:EE1 chromatographiert. Man erhielt 1g (100%) reines Produkt.
¹H-NMR (CDCl₃): 6.80-6.77 (m, 3H); 4.27 (s, 4H); 3.78 (dd, J=2.61, 4.02, 1H); 3.11 (dd, J=4.02, 5.4, 1H); 2.79 (dd, J=2.61, 4.5, 1H)

### Synthese von (R,S)-1-(2,3-Dihydro-benzo[1,4]dioxin-6-yl)-2-[4-(7-methoxy-naphthalen-1-yloxymethyl)-piperidin-1-yl]-ethanon

Zu einer Lösung von 4-(7-Methoxy-naphthalen-1-yloxymethyl)-piperidin (100mg, 0.36 mmol) und 6-Oxiranyl-2,3-dihydro-benzo[1,4]dioxin (66 mg; 0.36 mmol) in DMF (1 ml) wurde Lithiumperchlorat (39.2 mg; 0.36 mmol) und Kaliumcarbonat (101.9 mg; 0.73 mmol) zugegeben. Das Reaktionsgemisch wurde über Nacht bei 80 C gerührt, am Hochvakuum eingeengt, in Wasser aufgenommen und mit EtOAc extrahiert. Die organische Phase wurde über Na₂SO₄ getrocknet und eingeengt. Das Produkt wurde mittels Chromatographie an SiO₂ (EtOAc) gereinigt. Man erhielt 62.5 mg (38%) als beigen Schaum.
MS (ESI⁺) 450.5 [M+H⁺]

### Beispiel 3: (R,S)-6-{1-Hydroxy-2-[4-(7-methoxyphthalazin-1-yloxymethyl)-piperidin-1-yl]-ethyl}-4H-benzo[1,4]oxazin-3-on

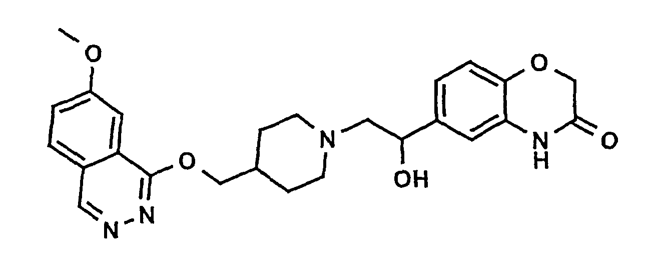

### Synthese von 1-Chloro-7-methoxy-phthalazin

Ein Gemisch aus 7-Methoxy-2H-phthalazin-1-on (2.2 g, 12.5 mmol, hergestellt wie in J. Am. Chem. Soc 1924, 1889 beschrieben) und POCl₃ (10 ml) wurde während 6h zum Rückfluss erhitzt. Der Überschuss POCl₃ wurde am Rotationsverdampfer abdestilliert und der Rückstand in EtOAc aufgenommen. Organische Phase mit Wasser und Bicarbonatlösung gewaschen, über MgSO₄ getrocknet und eingeengt. Das Produkt wurde mittels Säulenchromatographie (hex/EtOAc 1:1) gereinigt.
¹H-NMR (CDCl₃) : 9.33 (s, 1H) ; 7.92 (d, J=8.7 Hz, 1H) ; 7.58 (dd, J=8.7, 2.2 Hz, 1H); 7.52 (d, J=2.2 Hz, 1H); 4.0 (s, 3H)
MS (ESI⁺) 195/197 [M+H⁺]

### Synthese von 4-(7-Methoxy-phthalazin-1-yloxymethyl)-piperidin-1-carbonsäure tert-butyl ester

Eine Lösung von 4-Hydroxymethyl-piperidin-1-carbonsäure tert-butyl ester (475 mg, 2.2 mmol) in DMF (10 ml) wurde mit NaH Dispersion (55%, 96 mg) versetzt und 5 Minuten rühren gelassen. Eine Lösung von 1-Chloro-7-methoxyphthalazine (430 mg; 2.2 mmol) in DMF wurde zugetropft und das Reaktionsgemisch während 4h bei Raumtemperatur gerührt, mit EtOAc und Wasser verdünnt. Die organische Phase wurde mit Wasser gewaschen und über MgSO4 getrocknet und eingeengt. Das Produkt wurde mittles Chromatogrphie an SiO₂ (EtOAc) gereinigt. Es wurden 709 mg (86%) erhalten.
MS (ESI⁴) 374.5 [M+H⁺]

### Synthese von 7-Methoxy-2-(piperidin-4-ylmethoxy)-phthalazin

Die BOC gruppe wurde analog Beispiel 1 mit TFA in DCM entschützt.
MS (ESI⁺) 284.5 [M+H⁺]

Synthese von 6-{2-[4-(7-Methoxy-phthalazin-1-yloxymethyl)-piperidin-1-yl]-acetyl}-4H-benzo[1,4]oxazin-3-on Ein Gemisch aus 7-Methoxy-1-(piperidin-4-ylmethoxy)-phthalazin (273 mg, 1 mmol) und 6-(2-Chloro-acetyl)-4H-benzo[1,4]oxazin-3-on (225 mg, 1 mmol) in THF (5 ml) wurde mit Triethylamin (1 ml) versetzt und während 2h auf 50 C erhitzt. Es bildete sich ein gelber Niederschlag, der abgenutscht und mit MeOH/EtOH/THF verrührt wurde. Man erhielt auf diese Weise 80 mg reines Produkt.
MS (ESI⁺) 463.5 [M+H⁺]

### Synthese von (R,S)-6-{1-Hydroxy-2-[4-(7-methoxyphthalazin-1-yloxymethyl)-piperidin-1-yl]-ethyl}-4H-benzo[1,4]oxazin-3-on

Eine Lösung von 6-{2-[4-(7-Methoxy-phthalazin-1-yloxymethyl)-piperidin-1-yl]-acetyl}-4H-benzo[1,4]oxazin-3-on (40 mg) in EtOH (2 ml) und THF (2 ml) wurde mit NaBH₄ (1 eq) versetzt und während 2h bei rt gerührt. Das Reaktionsgemisch wurde auf SiO₂ adsorbiert und mittels Chromatographie (DCM/MeOH 9:1 +1% NH4OH) gereinigt. Man erhielt 25 mg reines Produkt.
MS (ESI⁺) 465.5 [M+H⁺]

### Beispiel 4: (R,S)-1-(2,3-Dihydro-benzo[1,4]dioxin-6-yl)-2-[4-(7-methoxy-phthalazin-1-yloxymethyl)-piperidin-1-yl]-ethanol

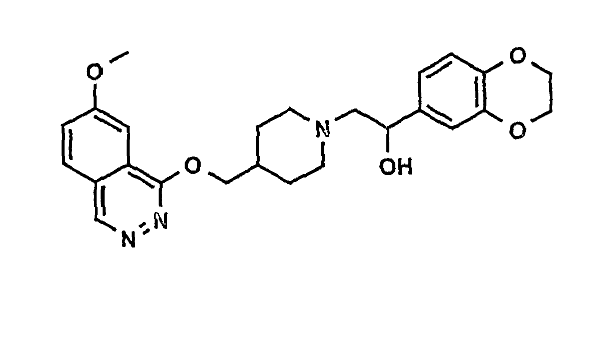

Zu einer Lösung von 7-Methoxy-1-(piperidin-4-ylmethoxy)-phthalazin (100mg, 0.36 mmol) und 6-Oxiranyl-2,3-dihydrobenzo[1,4]dioxin (66 mg; 0.36 mmol) in DMF (1 ml) wurde Lithiumperchlorat (39.2 mg; 0.36 mmol) und Kaliumcarbonat (101.9 mg; 0.73 mmol) zugegeben. Das Reaktionsgemisch wurde über Nacht bei 80 C gerührt, am Hochvakuum eingeengt, in Wasser aufgenommen und mit EtOAc extrahiert. Die organische Pahse wurde über Na₂SO₄ getrocknet und eingeengt. Das Produkt wurde mittels Chromatographie an SiO₂ (EtOAc) gereinigt. Man erhielt 58.7 mg (35%) als weissen Schaum.
MS (ESI⁺) 452.5 [M+H⁺]

### Beispiel 5: 6-{1-Hydroxy-2-[4-(7-methoxy-isochinolin-1-yloxymethyl)-piperidin-1-yl]-ethyl}-4H-benzo[1,4]oxazin-3-on

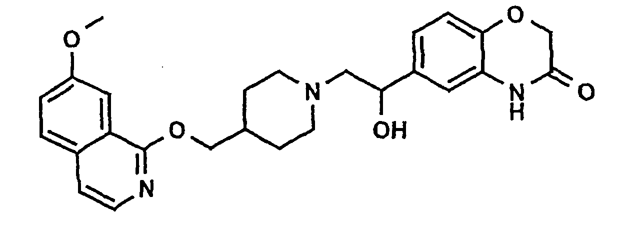

### Synthese von 1-Chloro-7-methoxy-isochinolin

Ein Gemisch aus 7-Methoxy-2H-isochinolin-1-on (6.5 g, 37 mmol, hergestellt analog J. Heterocycl. Chem 1985, 22, 328) und POCl₃ (50ml) wurde während 6h zum Rückfluss erhitzt. Der Überschuss POCl₃ wurde am Rotationsverdampfer abdestilliert und der Rückstand in EtOAc aufgenommen. Organische Phase mit Eiswasser und Bicarbonatlösung gewaschen, über MgSO₄ getrocknet und eingeengt. Das Produkt wurde mittels Säulenchromatographie (hex/EtOAc 3:1) gereinigt.
MS (ESI⁺) 194.5 [M+H⁺]

### Synthese von 4-(7-Methoxy-isochinolin-1-yloxymethyl)-piperidin-1-carbonsäure tert-butyl ester

Eine Lösung von 4-Hydroxymethyl-piperidin-1-carbonsäure tert-butyl ester (1075 mg, 5 mmol) in THF (20 ml) wurde mit NaH Dispersion (55%, 240 mg) versetzt und 5 Minuten rühren gelassen. Eine Lösung von 1-Chloro-7-methoxyisochinolin (965 mg; 5 mmol) in THF wurde zugetropft und das Reaktionsgemisch während 5h bei 50C und über Nacht bei Raumtemperatur gerührt, mit Ether und Wasser verdünnt. Die organische Phase wurde mit Wasser gewaschen und über MgSO₄ getrocknet und eingeengt. Das Produkt wurde mittles Chromatographie an SiO₂ (hex/EtOAc 3:1) gereinigt. Es wurden 1.16 g (62%) erhalten.
MS (ESI⁺) 373.5 [M+H⁺]

### Synthese von 7-Methoxy-1-(piperidm-4-ylmethoxy)-isochinolin

Die BOC gruppe wurde analog Beispiel 1 mit TFA in DCM entschuetzt.
¹H-NMR (CDCl₃): 7.8 (d, J=5.97 Hz, 1H); 7.58 (d, J=8.91, 1H); 7.43 (d, J=2.52, 1H); 7.24, (dd, J=8.91, 2.52, 1H); 7.08 (d, J=5.97, 1H) ; 4.32 (d, J=6.51, 2H) ; 3.88 (s, 3H) ; 3.26-3.24 (m, 2H); 2.88-2.70 (m, 2H); 2.1-2.05 (m, 1H); 2.0-1.9 (m, 2H); 1.60-1.46 (m, 2H)
Synthese von 6-{2-[4-(7-Methoxy-isochinolin-1-yloxymethyl)-piperidin-1-yl]-acetyl}-4H-benzo[1,4]oxazin-3-on Ein Gemisch aus 7-Methoxy-1-(piperidin-4-ylmethoxy)-isochinolin (272 mg, 1 mmol) und 6-(2-Chloro-acetyl)-4H-benzo[1,4]oxazin-3-on (225 mg, 1 mmol) in THF (5 ml) wurde mit K₂CO₃ (1 eq) versetzt und über Nacht auf 50 C erhitzt. Das Reaktionsgemisch wurde eingeengt und der Rückstand mittels Chromatographie an SiO₂ (EtOAc) gereinigt. Man erhielt auf diese Weise 250 mg (54%) reines Produkt.
MS (ESI⁺) 462.5 [M+H⁺]

### Synthese von 6-{1-Hydroxy-2-[4-(7-methoxy-isochinolin-1-yloxymethyl)-piperidin-1-yl]-ethyl}-4H-benzo[1,4]oxazin-3-on

Eine Lösung von 6-{2-[4-(7-Methoxy-isochinolin-1-yloxymethyl)-piperidin-1-yl]-acetyl}-4H-benzo[1,4]oxazin-3-on (200 mg, 0.5 mmol) in EtOH (20 ml) wurde mit NaBH₄ (40 mg) versetzt und während 2h bei Raumtemperatur gerührt. Das Reaktionsgemisch wurde auf SiO₂ adsorbiert und mittels Chromatographie (DCM/MeOH 9:1 +1% NH40H) gereinigt. Das Produkt wurde aus Ether kristallisiert. Man erhielt 55 mg (28%) reines Produkt.
MS (ESI⁺) 464 [M+H⁺]

### Beispiel 6: Synthese von 1-(2,3-Dihydro-benzo[1,4]dioxin-6-yl)-2-[4-(7-methoxy-isochinolin-1-yloxymethyl)-piperidin-1-yl]-ethanol

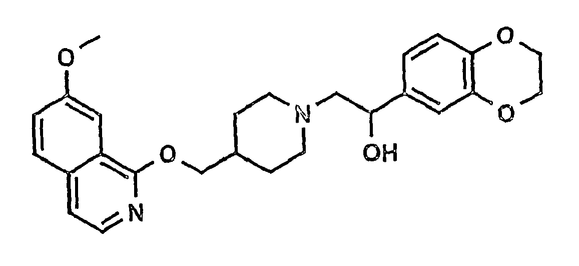

Zu einer Lösung von 7-Methoxy-1-ipiperidin-4-ylmethoxy)-isochinolin (100mg, 0.36 mmol) und 6-Oxiranyl-2,3-dihydro-benzo[1,4]dioxin (66 mg; 0.36 mmol) in DMF (1 ml) wurde Lithiumperchlorat (39.2 mg; 0.36 mmol) und Kaliumcarbonat (101.9 mg; 0.73 mmol) zugegeben. Das Reaktionsgemisch wurde über Nacht bei 80 C gerührt, am Hochvakuum eingeengt, in Wasser aufgenommen und mit EtOAc extrahiert. Die organische Phase wurde über Na₂SO₄ getrocknet und eingeengt. Das Produkt wurde mittels Chromatographie an SiO₂ (EtOAc) gereinigt. Man erhielt 58.7 mg (35%) als weissen Schaum.
MS (ESI⁺) 451.5 [M+H⁺]

### Beispiel 7: 2-(3-{[(2,3-Dihydro-benzo[1,4]dioxin-6-yl-methyl)-amino]-methyl}-piperidin-1-yl)-1-(3-methoxychinolin-5-yl)-ethanol

### Synthese von 3-Azidomethyl-piperidin-1-carbonsäure tert-butyl ester

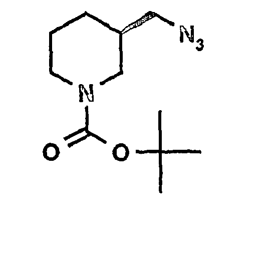

Zu einer Lösung von (3R)-Hydroxymethyl-piperidin-1-carbonsäure tert-butyl ester (2g, 9.29 mmol hergestellt analog Tetrahedron Lett 2002, 43, 8917 und Gazz. Chim. Ital. 1972, 102, 189.) in DCM (30 mL) wurden bei 0C, Triethylamin (2.6mL, 18.6mmol), und dann Methansulfonylchlorid (0.8mL, 10.3 mmol) zugetropft. Das Reaktionsgemisch wurde während 30 min bei dieser Temperatur gerührt. Gesättigte NaHCO₃ Lösung (20mL) und DCM (30mL) wurden zugegeben. Die zwei Phasen wurden getrennt und die organische Phase mit Sole (20mL) gewaschen, über MgSO₄ getrocknet und eingeengt. Das Rohprodukt wurde schnell über Silica filtriert (AcOEt/Hex 1:1.) . Das Rohprodukt wurde in DMF (40 ml) aufgenommen und mit Natriumazid (1.2 g, 18.4 mmol) versetzt. Das Reaktionsgemisch wurde während 5h bei 80°C gerührt, am Rotationsverdampfer eingeengt und mit Ether und Wasser versetzt. Die organische Phase wurde über MgSO₄ getrocknet und eingeengt. Das Rohprodukt wurde mittels Chromatographie an SiO₂ (hex/EtOAc 4:1) gereinigt. Es konnten 2.16g (9 mmol) als Oel isoliert werden.
MS (EI) m/z : 241.4 [M+H⁺]

### Synthese von (R)-(2,3-Dihydro-benzo[1,4]dioxin-6-yl-methyl)-piperidin-3-ylmethylamin

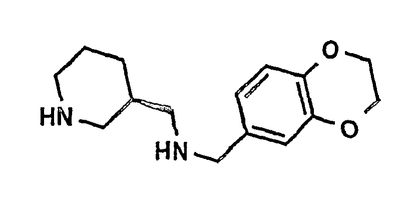

Eine Lösung von 3-Azidomethyl-piperidin-1-carbonsäure tert-butyl ester (2.16 g, 9 mmol) in THF (60mL) und Wasser (1mL) wurde mit polymergebundenem Triphenylphosphin (6.3 g, 3.6 mmol/g) versetzt. Das Gemisch wurde während 4 Tagen bei Raumtemperatur gerührt und filtriert. Das Filtrat wurde eingeengt und in Methanol (35ml) aufgenommen. 1,4-Benzodioxan-6-carboxaldehyde (1.48 g, 9 mmol) und 3A MS (9.6g) wurden zugegeben. Das Reaktionsgemisch wurde während 5h bei Raumtemperatur gerührt, bevor Natriumborhydrid (1.2g, 31.7 mmol) zugegeben wurde. Das Gemisch wurde für weitere 16h bei Raumtemperatur gerührt, eingeengt und in Wasser (100ml) aufgenommen. Die wässrige Phase wurde mit DCM (2*200ml) extrahiert. Die vereinten organischen Phasen wurden über MgSO₄ getrocknet und eingeengt. Der Rückstand wurde mittles Chromatographie an SiO₂ (DCM-MeOH 19-1) gereinigt. Es wurden 2.2 g Produkt als Oel erhalten. Dieses Oel wurde in TFA (10 ml) aufgenommen und während 1 h gerührt. Das Gemisch wurde eingeengt, in wässriger Ammoniaklösung aufgenommen und mit DCM (2*30ml) extrahiert. Die vereinten organischen Phasen wurden über MgSO₄ getrocknet und eingeengt. Es konnten 1.44g (5.53 mmol) Produkt als Oel isoliert werden.
MS (EI) m/z : 263.0 [M+H⁺]

### Synthese von 2-Bromo-1-(3-methoxy-chinolin-5-yl)-ethanon (Synthesis 2002, 83)

Eine Lösung aus 3-Bromchinolin (1.0.4 g, 50 mmol) in conc. H₂SO₄ (50 ml) wurde bei Raumtemperatur mit NBS (10.7g, 60 mmol) versetzt und über Nacht gerührt. Das Reaktionsgemisch wurde auf Eis gegossen, mit wässriger Ammoniaklösung basisch gestellt und mit Ether extrahiert. Die organischen Phasen wurden über MgSO₄ getrocknet und eingeengt. Das Produkt wurde mittels Chromatographie an SiO₂ (DCM/hex 6:4, DCM, EtOAc) gereinigt und aus Methanol umkristallisiert. Man erhielt 8g (56%) 3,5-Dibromochinolin als weisse Kristalle.
1H-NMR (CDCl3): 8.91 (d, J=2.2 Hz, 1H); 8.80 (d, J=2.2 Hz, 1H); 8.07 (d, J=7.8 Hz, 1H); 7.88 (d, J=7.8 Hz, 1H);7.60 (t, J=7.8Hz, 1H);
MS (ESI+) m/z 285/287/289 [M+H⁺]

Dieses Dibromid (2 mmol) wurde durch Reaktion mit Natriummethylat (4 mmol) in HMPT (8 ml) (Tetrahedron 2002, 58, 1125) gelöst und im Mikrowellenofen während 2 min auf 90 C erhitzt. Diese Prozedur wurde 6mal wiederholt. Das vereinte Reaktionsgemisch wurde auf Wasser gegossen, mit Ether extrahiert, über MgSO₄ getrocknet und eingeengt. Das Produkt wurde mittles Chromatographie ans SiO₂ (hex/EtOAc 4:1) gereinigt. Man erhielt 2.78 g (67%) 5-Bromo-3-methoxychinolin.

Dieses 5-Bromo-3-methoxychinolin wurde zu 1-(3-Methoxychinolin-5-yl)-ethanon umgesetzt wie in der Literatur (WO 0208224) beschrieben.

1-(3-Methoxy-chinolin-5-yl)-ethanon (500 mg, 2.5 mmol) in AcOH (10 ml) wurde mit Br₂ (1eq) und HBr (33% in AcOH) versetzt. Das Gemisch wurde während 2h bei rt gerührt. Laut MS bildete sich ein Gemisch aus mono- und dibromierten Produkt. Das Reaktionsgemisch wurde mit Wasser verdünnt und mit DCM extrahiert. Organische Phasen mit Wasser und Bicarbonatlösung gewaschen, über MgSO₄ getrocknet und eingeengt. Die Produkte wurden mittels Chromatographie an SiO₂ (hex/EtOAc 2:1) getrennt. Man erhielt 225 mg 2-Bromo-1-(3-methoxy-chinolin-5-yl)-ethanon.
1H-NMR (CDC13): 8.75 (d, J=2.2 Hz, 1H); 8.65 (d, J=2.2 Hz, 1H); 8.33 (d, J=7.8 Hz, 1H); 8.13 (d, J=7.8 Hz, 1H); 7.64 (t, J=7.8Hz, 1H); 4.65 (s, 2H); 4.01 (s, 3H).
MS (ESI+) m/z 280/282 [M+H⁺]

### Synthese von (1-RS)-2-(3(S)-{[(2,3-Dihydro-benzo-[1,4]-dioxin-6-ylmethyl)-amino]-methyl}-piperidin-1-yl)-1-(3-methoxy-chinolin-5-yl)-ethanol

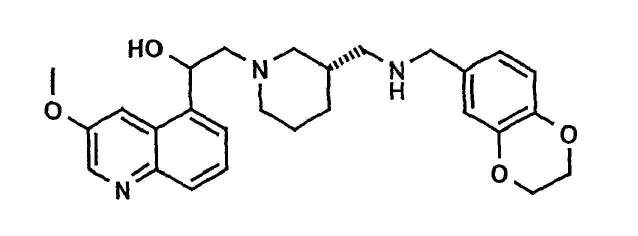

Eine Lösung von 2-Bromo-1-(3-methoxy-chinolin-5-yl)-ethanon (0.113 g, 0.4 mmol) und (R)-(2,3-Dihydro-benzo[1,4]dioxin-6-ylmethyl)-piperidin-3-ylmethyl-amin (0.106 g, 0.4 mmol) in THF (3mL) wurde über Nacht bei Raumtemperatur gerührt. Das Reaktionsgemisch wurde eingeengt und der Rückstand in Methanol (2mL) aufgenommen. Nachdem auf 0°C gekühlt worden war, wurde NaBH₄ (0.031g, 0.8 mmol) zugegeben. Das Reaktionsgemich wurde während einer Stunde bei 0°C gerührt. Es wurde Wasser (3 ml) zugegeben und das Reaktionsgemisch eingeengt. Der Rückstand wurde mittels Chromatographie (DCM-MeOH 9-1 1% NH₄OH) gereinigt. Man erhielt (1-RS)-2-(3(S)-{[(2,3-Dihydro-benzo[1,4]dioxin-6-ylmethyl)-amino]-methyl}-piperidin-1-yl)-1-(3-methoxy-chinolin-5-yl)-ethanol (0.097g, 0.21 mmol).
MS (EI) m/z: 464.5 [M+H⁺]

Die folgenden Beispiele wurden analog zu den oben beschriebenen hergestellt: 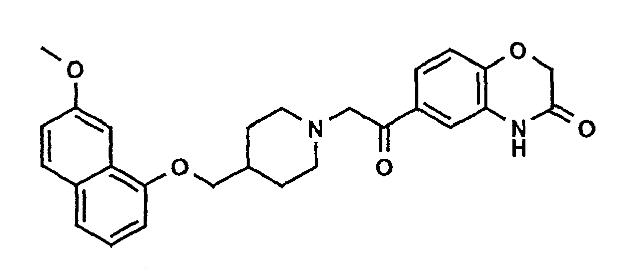 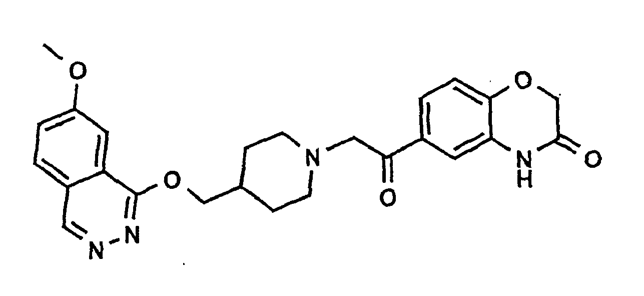 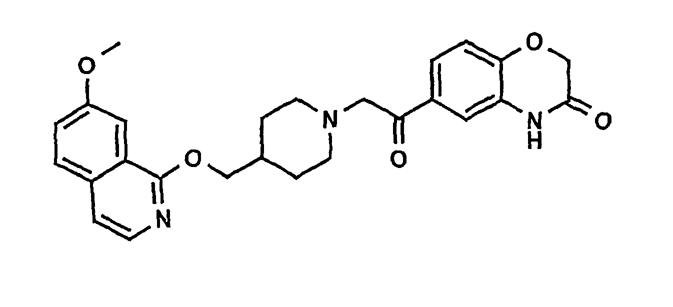 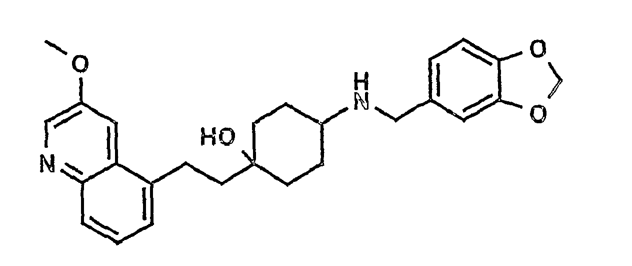 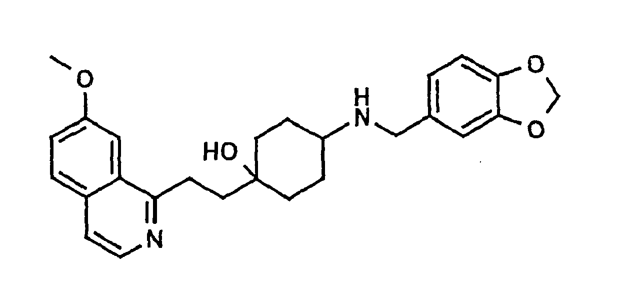 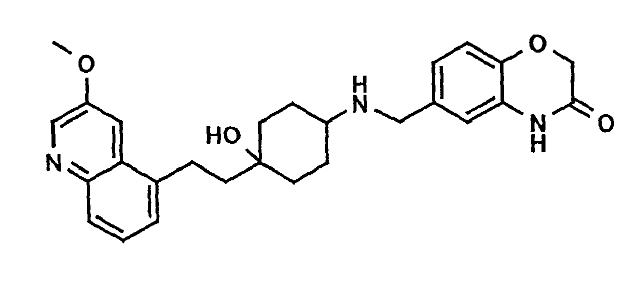
1. Verbindungen der Formel (I): 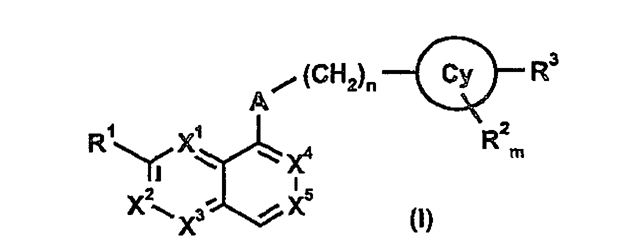 wobei
   A ein Sauerstoff- oder ein Schwefelatom, eine NH, eine Alkylen-, eine Alkenylen-, eine Alkinylen- oder eine Heteroalkylengruppe ist,
   X¹, X², X³, X⁴ und X⁵ unabhängig voneinander Stickstoffatome oder Gruppen der Formel CH oder CR⁴ sind,
   Cy eine Cycloalkylen-, eine Heterocycloalkylen-, eine Arylen- oder eine Heteroarylengruppe ist.
   R¹ ein Wasserstoffatom, ein Halogenatom, eine Hydroxy-, eine Amino-, eine Thiol-, eine Alkyl-, eine Heteroalkyl-, eine Alkyloxy-, eine Heteroalkyloxy-, eine Cyloalkyl-, eine Heterocycloalkyl-, eine Alkylcycloalkyl-, eine Heteroalkylcycloalkyl-, eine Cycloalkyloxy-, eine Alkylcycloalkyloxy-, eine Heterocycloalkyloxy oder eine Heteroalkylcycloalkyloxygruppe ist, die Reste R² unabhängig voneinander ein Halogenatom, eine Hydroxy-, Amino-, Nitro- oder Thiolgruppe, ein Alkyl-, Alkenyl-, Alkinyl-, Heteroalkyl-, Aryl-, Heteroaryl-, Cycloalkyl-, Alkylcycloalkyl-, Heteroalkylcycloalkyl-, Heterocycloalkyl-, Aralkyl- oder ein Heteroaralkylrest sind, oder zwei der Reste R² zusammen Teil eines Aryl-, Heteroaryl-, Cycloalkyl-, Heterocycloalkyl-, Alkylcycloalkyl-, Heteroalkylcycloalkyl-, Aralkyl- oder ein Heteroaralkylrings sind,
   R³ ein Alkyl-, Alkenyl-, Alkinyl-, Heteroalkyl-, Aryl-, Heteroaryl-, Cycloalkyl-, Alkylcycloalkyl-, Heteroalkyl-cycloalkyl-, Heterocycloalkyl-, Aralkyl- oder ein Heteroaralkylrest ist,
   R⁴ ein Halogenatom, eine Hydroxy-, Alkyl-, Alkenyl-, Alkinyl- oder eine Heteroalkylgruppe ist,
   n gleich 0, 1 oder 2 ist und
   m gleich 0, 1 oder 2 ist,
   oder ein pharmakologisch akzeptables Salz, Solvat, Hydrat oder eine pharmakologisch akzeptable Formulierung derselben.
2. Verbindungen nach Ausführungsform 1, wobei A ein Sauerstoff- oder ein Schwefelatom oder eine Gruppe der Formel CH₂, CH₂CH₂, CH₂N(C₁-C₄-Alkyl) , N(C₁-C₄-Alkyl)CH₂, CH₂O, OCH₂, CH₂S, SCH₂ , CH₂CH(OH), CH(OH), CH(OH)CH₂, NHCO, CONH, C(=O)CH₂ oder CH₂C(=O) ist.
3. Verbindungen nach Ausführungsform 1 oder 2, wobei drei, vier oder fünf der Gruppen X¹, X², X³, X⁴ und X⁵, CH-Gruppen sind.
4. Verbindungen nach einer der Ausführungsformen 1 bis 3, wobei R¹ eine C₁-C₄-Alkyloxy- oder eine C₁-C₄-Heteroalkyloxygruppe ist, wobei ein oder mehrere Wasserstoffatome dieser Gruppen durch Fluoratome ersetzt sein können.
5. Verbindungen nach einer der Ausführungsformen 1 bis 3, wobei R¹ eine Methoxygruppe ist.
6. Verbindungen nach einer der Ausführungsformen 1 bis 5, wobei R² eine Hydroxy-, eine C₁-C₄-Alkyl-, C₁-C₄-Heteroalkyl- oder eine C₆-C₁₂-Heteroaralkylgruppe ist.
7. Verbindungen nach einer der Ausführungsformen 1 bis 6, wobei R³ eine Heteroalkylcycloalkyl- oder eine Heteroaralkylgruppe ist.
8. Verbindungen nach einer der Ausführungsformen 1 bis 6, wobei R³ eine Gruppe der Formel -B-Y ist, wobei B eine Alkylen-, Alkenylen-, Alkinylen- oder eine Heteroalkylengruppe ist und Y eine Aryl-, Heteroaryl-, Aralkyl-, Heteroaralkyl-, Cycloalkyl-, Heterocycloalkyl-, Alkylcycloalkyl- oder eine Heteroalkylcycloalkylgruppe ist.
9. Verbindungen nach Ausführungsform 8, wobei Y eine der folgenden Strukturen aufweist, 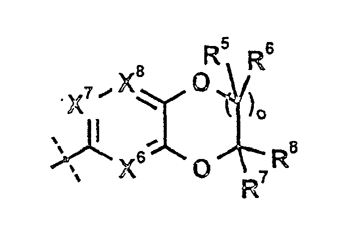 oder 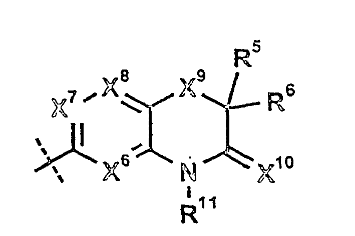 wobei X⁶, X⁷ und X⁸ unabhängig voneinander Stickstoffatome oder Gruppen der Formel CR⁹ sind, X⁹ und X¹⁰ unabhängig voneinander Sauerstoff- oder Schwefelatome oder Gruppen der Formel NR¹⁰ sind, o gleich 0, 1 oder 2 ist, R⁵, R⁶, R⁷, R⁸ und R⁹ unabhängig voneinander Wasserstoffatome, Halogenatome, Hydroxy-, Alkyl-, Alkenyl-, Alkinyl- oder Heteroalkylgruppen sind und R¹⁰ und R¹¹ unabhängig voneinander Wasserstoffatome, Alkyl-, Alkenyl-, Alkinyl- oder Heteroalkylgruppen sind.
10. Verbindungen nach Ausführungsform 8, wobei Y eine der folgenden Strukturen aufweist: 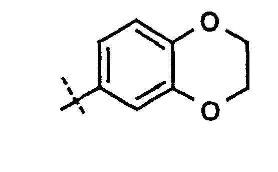 oder 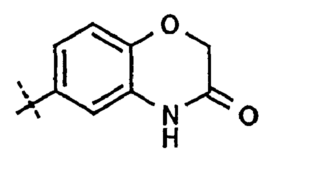
11. Verbindungen nach einer der Ausführungsformen 1 bis 10, wobei der Linker -A-(CH₂)- eine Kettenlänge von 2 oder 3 Atomen aufweist.
12. Verbindungen nach einer der Ausführungsformen 1 bis 11, wobei R⁴ ein Fluor- oder ein Chloratom oder eine C₁-C₄-Alkyloxy-, oder eine C₃-C₆-Dialkylaminomethylgruppe ist, wobei ein oder mehrere Wasserstoffatome dieser Gruppen durch Fluoratome ersetzt sein können.
13. Verbindungen nach einer der Ausführungsformen 1 bis 12, wobei Cy eine Cycloalkylen- oder eine Heterocycloalkylengruppe mit ein oder zwei Ringen und 4, 5, 6, 7, 8, 9 oder 10 Ringatomen ist.
14. Verbindungen nach einer der Ausführungsformen 1 bis 12, wobei Cy eine der folgenden Strukturen aufweist, 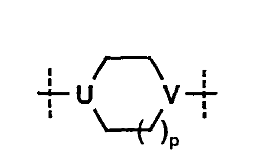 oder 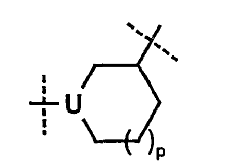 wobei U ein Stickstoffatom oder eine Gruppe der Formel CH oder COH ist und V ein Stickstoffatom oder eine CH-Gruppe ist und p gleich 0 oder 1 ist.
15. Pharmazeutische Zusammensetzungen, die eine Verbindung nach den Ausführungsformen 1 bis 14 als Wirkstoff und fakultativ Trägerstoffe und/oder Adjuvanzien enthalten.
16. Verwendung einer Verbindung oder einer pharmazeutischen Zusammensetzung nach einer der Ausführungsformen 1 bis 15 zur Behandlung von Bakterieninfektionen.

## Patentansprüche

1. Verbindungen der Formel (I): 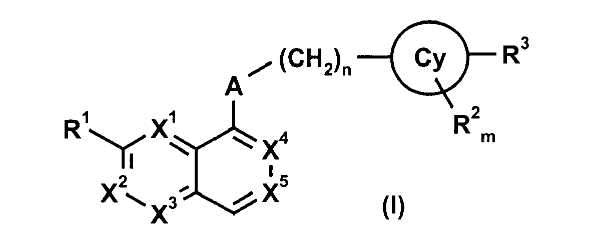 wobei
A ein Sauerstoff- oder ein Schwefelatom oder eine Gruppe der Formel CH₂, CH₂CH₂, CH₂N(C₁-C₄-Alkyl), N(C₁-C₄-Alkyl) CH₂, CH₂O, OCH₂, CH₂S, SCH₂, CH₂CH(OH) , CH (OH) , CH(OH)CH₂, NHCO, CONH, C(=O)CH₂ oder CH₂C(=O) ist und der Linker -A-(CH₂)ₙ- eine Kettenlänge von 2 oder 3 Atomen aufweist,
X¹, X², X³, X⁴ und X⁵ unabhängig voneinander Stickstoffatome oder Gruppen der Formel CH oder CR⁴ sind,
Cy eine Cycloalkylen- oder eine Heterocycloalkylengruppe mit ein oder zwei Ringen und 4, 5, 6, 7, 8, 9 oder 10 Ringatomen ist,
R¹ eine C₁-C₄-Alkyloxy- oder eine C₁-C₄-Heteroalkyloxygruppe ist, wobei ein oder mehrere Wasserstoffatome dieser Gruppen durch Fluoratome ersetzt sein können,
die Reste R² unabhängig voneinander eine Hydroxy-, eine C₁-C₄-Alkyl-, C₁-C₄-Heteroalkyl- oder eine C₆-C₁₂-Heteroaralkylgruppe sind,
R³ eine Gruppe der Formel -B-Y ist, wobei B eine Alkylen-, Alkenylen-, Alkinylen- oder eine Heteroalkylengruppe ist und Y eine Aryl-, Heteroaryl-, Aralkyl-, Heteroaralkyl-, Cycloalkyl-, Heterocycloalkyl-, Alkylcycloalkyl- oder eine Heteroalkylcycloalkylgruppe ist,
R⁴ ein Fluor- oder ein Chloratom oder eine C₁-C₄-Alkyloxy-, oder eine C₃-C₆-Dialkylaminomethylgruppe ist, wobei ein oder mehrere Wasserstoffatome dieser Gruppen durch Fluoratome ersetzt sein können,
n gleich 0, 1 oder 2 ist und
m gleich 0, 1 oder 2 ist,
oder ein pharmakologisch akzeptables Salz, Solvat, Hydrat oder eine pharmakologisch akzeptable Formulierung derselben.

2. Verbindungen nach Anspruch 1, wobei drei, vier oder fünf der Gruppen X¹, X², X³, X⁴ und X⁵, CH-Gruppen sind.

3. Verbindungen nach Anspruch 1 oder 2, wobei R¹ eine Methoxygruppe ist.

4. Verbindungen nach einem der Ansprüche 1, 2 oder 3, wobei Y eine der folgenden Strukturen aufweist, 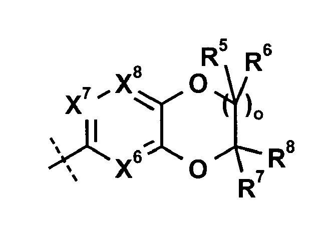 oder 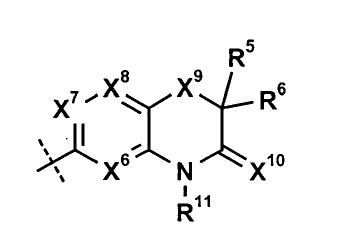 wobei X⁶, X⁷ und X⁸ unabhängig voneinander Stickstoffatome oder Gruppen der Formel CR⁹ sind, X⁹ und X¹⁰ unabhängig voneinander Sauerstoff- oder Schwefelatome oder Gruppen der Formel NR¹⁰ sind, o gleich 0, 1 oder 2 ist, R⁵, R⁶, R⁷, R⁸ und R⁹ unabhängig voneinander Wasserstoffatome, Halogenatome, Hydroxy-, Alkyl-, Alkenyl-, Alkinyl- oder Heteroalkylgruppen sind und R¹⁰ und R¹¹ unabhängig voneinander Wasserstoffatome, Alkyl-, Alkenyl-, Alkinyl- oder Heteroalkylgruppen sind.

5. Verbindungen nach einem der Ansprüche 1, 2 oder 3, wobei Y eine der folgenden Strukturen aufweist: 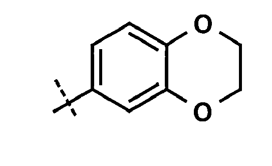 oder

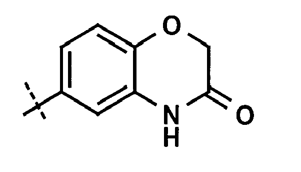

6. Verbindungen nach einem der Ansprüche 1 bis 5, wobei Cy eine der folgenden Strukturen aufweist, 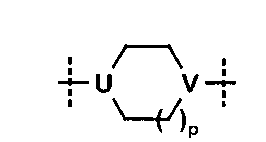 oder 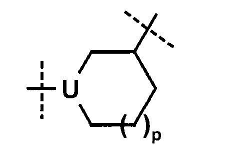 wobei U ein Stickstoffatom oder eine Gruppe der Formel CH oder COH ist und V ein Stickstoffatom oder eine CH-Gruppe ist und p gleich 0 oder 1 ist.

7. Pharmazeutische Zusammensetzungen, die eine Verbindung nach den Ansprüchen 1 bis 6 als Wirkstoff und fakultativ Trägerstoffe und/oder Adjuvanzien enthalten.

8. Verwendung einer Verbindung oder einer pharmazeutischen Zusammensetzung nach einem der Ansprüche 1 bis 7 zur Herstellung eines Medikaments zur Behandlung von Bakterieninfektionen.
